Europäisches Patentamt

European Patent Office

Office européen des brevets

(19)

(11) Numéro de publication: **0 155 730**
**A2**

## (12) DEMANDE DE BREVET EUROPEEN

(21) Numéro de dépôt: 85200328.4

(22) Date de dépôt: 07.03.85

(51) Int. Cl.⁴: **A 61 F 5/04**
**A 61 F 5/01**

(30) Priorité: 20.03.84 IT 6725984

(43) Date de publication de la demande:
25.09.85 Bulletin 85/39

(84) Etats contractants désignés:
AT BE CH DE FR GB LI LU NL SE

(71) Demandeur: GESTIONI RIUNITE TOSCANA GOMMA
S.p.A.
Via Palestro 101
I-27038 Robbio(IT)

(72) Inventeur: Pascale, Dott. Walter
Via F.lli Cervi Residenza Acquario
I-20100 Milanodue Milano(IT)

(72) Inventeur: Gilardi, Pier Enrico
Via Amedeo d'Aosta 18
I-27038 Robbio Pavia(IT)

(74) Mandataire: Patrito, Pier Franco, Dr. Ing.
Cabinet PATRITO BREVETTI Via Don Minzoni 14
I-10121 Torino(IT)

(54) Dispositif de contention pour l'usage dans l'orthopédie et dans la traumatologie.

(57) Un dispositif de contention destiné à être appliqué à des segments lésés de l'appareil squelettique afin de les immobiliser temporairement, formé par au moins deux coques (10,20) préformées en un matériau élastique, tel que du polyuréthane ou un matériau synthétique expansible similaire (polycast, etc). Chaque coque (10,20) a une forme générale qui reproduit substantiellement la forme de la parte à laquelle on doit appliquer le dispositif dans le but de réaliser la contention, et les coques sont prédisposées pour être assemblées au moyen de courroies (30) ou d'organes similaires, préférablement insérés dans des sièges (17,27) des coques (10,20), de sorte qu'on puisse les ouvirir ou les enlever. Des éléments d'armature, tels que des tissue, des attelles ou des matériaux de recouvrement, peuvent être incorporés dans le matériau des coques (10,20) pour les raidir. les coques peuvent éventuellement être adaptées aux measures du patient en les coupant (suivant la ligne 60) ou bien en superposant partiellement leurs bords. Ce dispositif peut être avantageusement utilisé en orthopédie, en traumatoglogie et en phisiothérapie de réhabilitation.

FIG. 1

## DISPOSITIF DE CONTENTION POUR L'USAGE DANS L'ORTHOPÈDIE ET DANS LA TRAUMATOLOGIE

La présente invention concerne un dispositif de contention destiné à être appliqué à des segments lésés de l'appareil squelettique afin de les immobiliser temporairement.

Dans l'orthopédie et dans la traumatologie se présente le problème d'immobiliser des parties de l'appareil squelettique, en cas de lésions telles que les fractures, les luxations, les lésions des ligaments et similaires, pour un temps suffisant à rétablir l'intégrité du segment squelettique lésé. Des problèmes similaires se posent parfois dans la physiothérapie de réhabilitation. Dans ce but on a recours, dans la plupart des cas, à des appareils plâtrés de différentes formes et dimensions. Ces appareils exercent leur fonction d'une manière satisfaisante en ce qui concerne l'immobilisation des segments squelettiques, mais ils présentent l'inconvénient de gêner, à cause de leur rigidité, tout mouvement musculaire, même le moindre, de sorte que les muscles des parties renfermées dans l'appareil plâtré risquent l'hypotrophie à cause du travail métabolique dynamique insuffisant. Il s'ensuit une considérable prolongation de la période successive de recouvrement fonctionnel, soit de la musculature intéressée que des articulations. De ce fait, dans beaucoup de cas l'emploi d'appareils plâtrés du type traditionnel donne lieu à des inconvénients même graves.

Pour limiter ces inconvénients on a proposé les ainsi-dits plâtres fonctionnels, c'est à dire des appareils de contention qui incorporent des moyens d'articulation disposés en correspondance des articulations squelettiques, dont ils permettent donc le mouvement. Cependant, ces appareils, ainsi que d'autres appareils proposés dans le même but, présentent quand-même une rigidité intrinsèque qui, en gênant les mouvements musculaires, mène, dans une mesure plus ou moins grande, auxdits phénomènes d'hypotrophie. En outre, ces appareils ne sont pas indiqués pour le traitment de certains types de lésions. De plus, un inconvé-

nient commun à tous les types d'appareils plâtrés réside dans la perte de temps et l'incommodité pour le patient, inhérentes aux opérations de formation et de démolition de l'appareil. Un autre inconvénient des appareils plâtrés connus consiste en ce que pour chaque accès d'inspection ou pansement aux parties lésées il faut scier et puis reconstruire l'appareil, ce qui comporte des pertes de temps et des incommodités ultérieures pour le patient et pratiquement amène, dans plusieurs cas, à espacer les inspections par rapport à leur fréquence optimale.

Enfin, un inconvénient général des appareils plâtrés est de présenter une radio-opacité considérable qui gêne les investigations radiologiques, lesquelles peuvent être effectuées en des conditions optimales seulement par l'enlèvement préalable de l'appareil.

Le but de la présente invention est de réaliser un dispositif de contention de conception nouvelle, capable de rémédier aux inconvénients mentionnés des appareils connus.

Ce but est atteint, selon l'invention, principalement par le fait que le dispositif de contention est formé par au moins deux coques préformées en matériau élastique, ayant une forme générale qui reproduit la forme de la partie à laquelle on doit appliquer le dispositif dans le but de réaliser la contention, et prédisposées pour être assemblées au moyen de courroies ou d'organes similaires de sorte qu'on puisse les ouvrir ou les enlever.

Grâce à ces caractéristiques, le dispositif de contention peut être appliqué à la partie à immobiliser, en empêchant tout déplacement des segments squelettiques, mais en contenant la musculature d'une façon souple, de sorte à ne pas gêner des mouvements musculaires limités, qui préviennent les phénomènes d'hypotrophie et permettent un recouvrement fonctionnel plus rapide.

Le caractère intrinsèque d'ouvrabilité ou de désassemblabilité du dispositif permet son application et son enlèvement par des opérations de courte durée et sans aucune incommodité pour le patient; elle permet en outre, à n'importe quel moment, l'ouverture ou l'enlèvement de l'une

des coques, tout en conservant l'effet de contention partielle exercé par l'autre coque, quand l'on désire d'effectuer une inspection et/ou un pansement des parties renfermées dans le dispositif. La même opération peut être aussi effectuée, dans certains cas, simplement dans le but de libérer temporairement les parties de l'action de contrainte exercée par le dispositif et pour en permettre l'aération, soit pour des raisons thérapeutiques que pour donner du soulagement au patient. Celle-ci est une possibilité caractéristique du dispositif selon l'invention et, dans certains cas, elle peut être d'une importance considérable.

Les coques formant le dispositif selon l'invention peuvent être réalisées en un matériau élastique généralement quelconque, mais dans beaucoup de cas resulte particulièrement avantageux l'emploi du polyuréthane ou d'un matériau synthétique expansible similaire, qui peut être soumis à des degrés d'expansion différents afin d'obtenir les caractéristiques de souplesse et d'élasticité générale qui sont estimées optimales pour chaque type d'application.

En outre, dans la masse de chaque coque, pour toute son extension ou seulement en des positions estimées appropriées, peuvent être incorporés des tissus ou des éléments similaires qui, en s'intégrant dans la structure du matériau synthétique, forment une armature qui permet d'obtenir une stabilité de forme préétablie, tout en utilisant un matériau ayant une souplesse supérieure à celle qui, par elle-même, donnerait lieu à la stabilité de forme désirée. On obtient ainsi un dispositif qui, bien qu'assurant le contention nécessaire, gêne encore moins les mouvements musculaires et resulte moins oppressif pour le patient. Un effet similaire peut être obtenu en incorporant dans le matériau constituant les coques, au lieu des tissus ou éléments similaires, ou bien en addition à ceux-ci, des attelles rigides ou semi-rigides s'étendant substantiellement dans la direction longitudinale des coques. Ces attelles peuvent être métalliques ou d'un matériau synthétique ayant une rigidité appropriée. Dans le moulage des coques ou peut aussi y incorporer des matériaux de revêtement, comme par exemple le synderme ou similaires, ayant un but esthétique mais qui peuvent aussi exercer la fonction d'une armature, en substitution ou en plus des moyens déjà mentionnés à cet effet. Enfin, dans les coques on peut encore incorporer, s'il est nécessaire, des éléments de soutien et/ou d'articulation, non directement destinés à la fonction de contention, mais à des buts accessoires, comme par exemple celui de permettre un appui efficace de la personne qui porte le dispositif. Dans tous les cas dans lesquels les coques ne présentent pas des pièces incorporées de nature métallique, le dispositif resulte radio-transparent et autorise l'exécution d'investigations radiologiques sans dérangements, sans qu'il soit nécessaire de démonter le dispositif.

Comme les coques formant le dispositif sont préformées avec une configuration reproduisant substantiellement celle de la partie du corps à laquelle le dispositif doit être appliqué, il est clair que pour satisfaire aux nécessités qui se présentent dans des cas différents d'applications on doit préformer dès coques de formes et de dimensions générales différentes, pour en constituer ainsi des assortiments dans lesquels on peut choisir chaque fois la pièce appropriée. Cependant, la nature du matériau utilisé pour la formation des coques, lequel se prête à être facilement coupé, permet un certain degré d'unification constructive, en choisissant des tailles relativement grandes dans la fabrication des coques et en procédant ensuite à une adaptation des coques, au moment de l'application, en enlevant des bandes de matériau d'au moins un bord d'une coque. Pour faciliter cette opération on peut éventuellement munir les coques elles-mêmes de graduations destinées à faciliter la définition des lignes de coupure. Alternativement, au moins un bord d'au moins une des coques peut être réalisé de sorte à pouvoir recevoir la superposition en mesure variable du bord correspondant d'une autre coque, en permettant ainsi une adaptation des dimensions globales du dispositif. Enfin, l'adaptation définitive aux formes du patient est effectuée, comme d'habitude, par l'interposition de couches ou de coussinets d'ouate o d'un matériau similaire entre la surface interne du dispositif et les parties à immobiliser.

Les caractéristiques et les avantages de l'objet de la présente invention ressortiront plus clairement de la suivante description de deux formes de réalisation, représentées schématiquement dans le dessin annexé, dans lequel:

La figure 1 est une vue en élévation latérale, à une échelle très réduite, d'un dispositif selon l'invention, formé par deux coques fabriquées séparément et juxtaposées;

les figures 2 et 3 montrent les deux coques formant le dispositif selon la figure 1, séparées et vues en plan du côté intérieur;

la figure 4 montre, à une échelle quelque peu plus grande, une section faite à travers le dispositif suivant la ligne IV-IV de la figure 1; et

la figure 5 montre, de façon similaire à la figure 4, une forme de réalisation modifiée du dispositif selon l'invention.

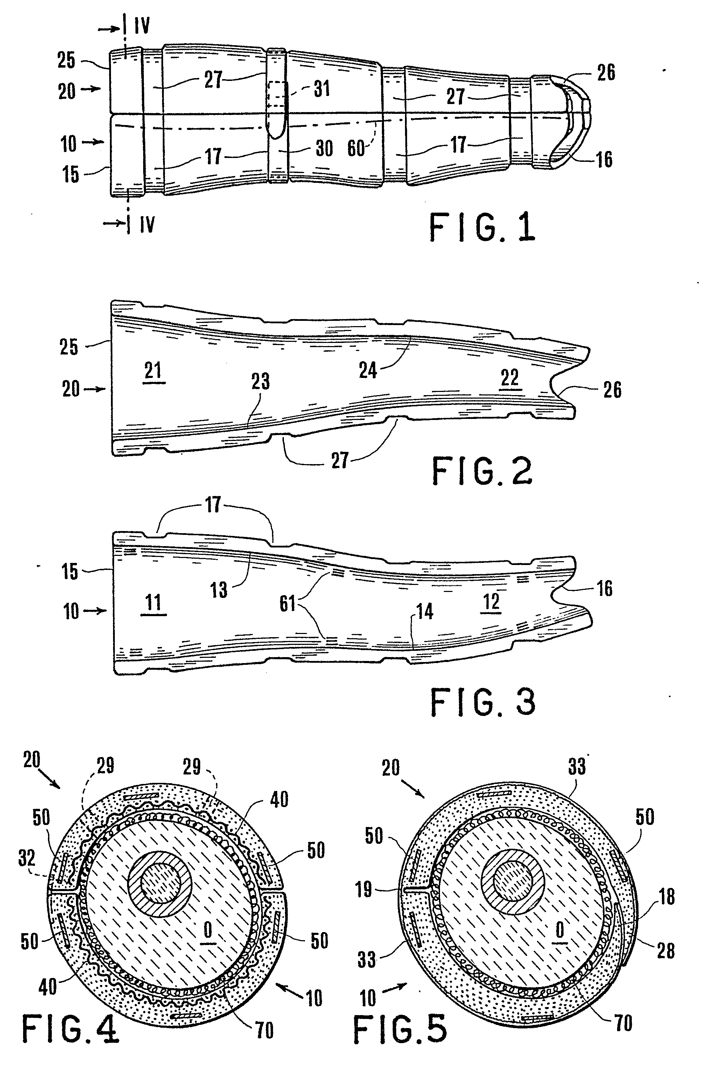

Dans la forme de réalisation représentée à titre d'exemple dans les figures 1 à 4, le dispositif selon l'invention est destiné à être utilisé pour l'immobilisation d'une jambe entre la cuisse et la cheville. Le dispositif comprend, dans cette forme de réalisation, deux coques constructivement séparées, indiquées généralement par 10 et 20, chacune desquelles à substantiellement la forme d'une tuile et a un profil qui suit généralement les formes d'une jambe, avec une partie d'extrémité 11, respectivement 21, d'un calibre plus grand, correspondant à la cuisse, et un effilement progressif vers la partie opposée de calibre plus petit 12, 22 correspondante à la jambe à l'endroit de la tibia immédiatement au-dessus de la cheville, le passage de l'une à l'autre extrémité ayant lieu, d'un côté, lelong d'une ligne concave 13, 23 correspondante à la convexité du genou suivie par une ligne convexe de raccordement, et de l'autre côté lelong d'une ligne approximativement droite suivie par un segment concave 14, 24 correspondant à la convexité du mollet. A' l'extrémité correspondante à la cuisse, les coques peuvent être limitées par une ligne substantiellement droite 15, 25, tandis que, à l'extrémité correspondante à la cheville, une ligne limite concave 16, 26 permet de

laisser libre l'articulation correspondante. Comme on le comprendra, la juxtaposition de deux coques comme celles indiquées par 10 et 20 en renfermant entr'elles une jambe 0, comme représenté schématiquement dans la figure 4, conduit à la formation d'une cavité contenant la jambe avec un jeu relativement limité, qui peut être éliminé convenablement au moyen de couches ou coussinets 70 d'ouate o d'un matériau similaire. Ensuite les deux coques, juxtaposées selon la figure 1, sont reliées au moyen de courroies circonférentielles. De préférance on peut prévoir pour ces courroies, sur la surface externe des coques 10 et 20, des sièges légèrement creusés, respectivement 17 et 27, grâce auxquels les courroies n' empèchent pas seulement l'éloignement réciproque des coques 10 et 20, mais aussi un glissement longitudinal réciproque accidentel.

Tout genre de courroie peut être utilisé pour relier les deux coques 10 et 20 mais, comme on n'a pas à prévoir des efforts particulièrement élevés, il est pratiquement avantageux d'utiliser des bandes 30 de tissu synthétique pourvues d'endroits 31 munis de saillies d'accrochage réciproque (ainsi-dit velcro), comme montré par exemple dans la figure 1.

La souplesse du matériau élastique formant les coques 1 et 2 est choisie d'une façon telle que les coques juxtaposées et reliées l'une l' autre soient capables de contenir le segment squelettique prévu d'une façon suffisante pour favoriser le rétablissement de son intégrité, mais qu'en même temps elles permettent des mouvements musculaires limités, dans le but spécifié dans le préambule. La souplesse du matériau doit donc varier en fonction de la nature de la partie à immobiliser. Dans les cas où une souplesse très limitée est nécessaire, on peut employer des matériaux élastiques massifs, mais dans maints cas il résulte particulièrement avantageux de fabriquer les coques en polyuréthane ou en un matériau synthétique similaire (par exemple l'ainsi-dit polycast), dont la souplesse peut être amplement ajustée en soumettant le matériau à un degré plus ou moins élevé d'expansion pendant son moulage. Dans les cas les plus simples, un choix approprié de la souplesse du matériau, ainsi que de l'épaisseur des coques, peut permettre de satisfaire d'une façon suffisante à ces exigences contrastantes.

Cependant, quand il paraît approprié, il est possible de régler la rigidité générale des coques et leur souplesse locale, indépendamment l'une de l'autre, en insérant dans les moules destinées a former les coques 10 et 20 des tissus, des filets ou des éléments similaires 40, destinés à s'imprégner de matériau synthétique et à être ensuite incorporés dans les coques formées. On peut employer des tissus élastiques, à l'effet d'un adaptement plus parfait aux formes des coques. Un choix approprié de la nature des tissus ou éléments similaires 40, de leur extension (qui peut intéresser la coque toute entière ou seulement une partie de celle-ci) et de leur position dans la section de la coque, permet de réaliser le raidissement général désiré de la coque, tout en maintenant à un degré élevé la souplesse du matériau formant la coque, de sorte à satisfaire de la meilleure façon possible aux exigences des coques destinées au dispositif selon l'invention.

Un effet similaire peut être obtenu en incorporant dans les coques 10 et 20 des attelles longitudinales 50 réalisées en un matériau de rigidité appropriée et dont la section a de préférance des côtés très différents, de sorte à influencer la rigidité de la coque dans des directions préférentielles. Ces attelles, en cas de nécessité, peuvent être aussi métalliques, mais en général elles peuvent être d'un matériau synthétique rigide ou semi-rigide, dans le but de ne pas réduire la radiotransparence du dispositif.

Naturellement, comme représenté dans la figure 4, les deux mesures d'incorporation de tissus et d'incorporation d'attelles peuvent coexister, si cela parait à propos.

Il est aussi possible de disposer dans les moules, lors du moulage des coques, des feuilles de matériau de revêtement, tel que du synderme ou du matériau similaire, lesquels forment ensuite la surface visible des coques, avec le résultat d'obtenir une amélioration esthétique et une protection contre le souillement. Ces matériaux de revêtement peuvent aussi assumer des fonctions d'armature, en plus ou en substitution

de celles exercées par les tissus ou par les attelles incorporés.

La possibilité d'incorporer des éléments étrangers dans la masse des coques formant le dispositif peut être utilisée aussi pour appliquer des organes ayant des fonctions accessoires différent de la véritable contention de la partie à immobiliser, comme par exemple des moyens de raidissement destinés à limiter la mobilité des articulations, des étriers pour l'appui du patient sur le terrain, etc.

Comme on peut le comprendre particulièrement de l'observation de la figure 4, quand le patient se trouve dans une position au moins approximativement horizontale, un effet partiel mais efficace d'immobilisation est exercé aussi par la seule coque inférieure 10, dans laquelle la jambe du patient trouve appui par gravité, ce qui offre la possibilité mentionnée d'enlever temporairement, sans risques, la coque supérieure 20, soit pour les inspections ou pour les pansements, que simplement pour aérer les parties du corps renfermées et donner du soulagement au patient.

Dans la figure 1 est indiquée une ligne en traits interrompus 60, s'étendant à proximité de la ligne de séparation entre les coques 10 et 20. On comprend que si par exemple la coque 10 est coupée lelong de la ligne 60 (ce qui peut être executé facilement en considération de la nature du matériau formant la coque, même dans le cas qu'elle incorpore des tissus d'armature), elle devient appropriée pour una jambe de longueur égale mais de dimensions différentes en grosseur, par rapport à la jambe pour laquelle la coque etait appropriée avant la coupure. Cette possibilité, associée à la possibilité de prévoir des épaisseurs variables dans certaines limites des coussinets d'ouate 70, permet de normaliser partiellement les dimensions constructives des coques préformées, sans en compromettre les fonctions. Pour faciliter la détermination de la ligne de coupure 60 correcte, en fonction de mesurations effectuées sur le patient, des graduations 61 peuvent être éventuellement executées sur la surface interne (ou externe) des coques, comme représenté par exemple dans la figure 3 pour la coque 10.

Dans l'exemple représenté, la partie à immobiliser est maintenue avec son articulation étendue, mais il est bien entendu que les coques du dispositif peuvent être configurées de façon correspondante à une position plus ou moins pliée d'une articulation, lors que cela est exigé.

Dans la forme de réalisation représentée dans les figures 1 à 4, les deux coques formant le dispositif sont constructivement séparées et fonctionnellement séparables, mais comme on peut le comprendre on obtient des effets similaires si les deux coques sont solidaires l'une de l'autre du fait qu'elles sont formées en une seule pièce ayant une partie formant charnière, ou bien qu'elles sont constructivement séparées mais ensuite reliées l'une l'autre au moyen d'une partie déformable formant charnière (32, figure 4), ou bien encore si elles sont en général tellement déformables qu'elle peuvent être ouvertes par déformation élastique. Dans ces cas on obtient une simplification de la construction et on assure positivement le positionnement réciproque correcte des coques dans la direction longitudinale.

Dans la forme de réalisation représentée dans la figure 5, les deux coques sont formées par deux parties d'une pièce constructivement unique, et elles sont unies à travers une partie 19 d'épaisseur réduite, agissant comme une charnière. En outre, comme représenté dans cette même figure 5, les bords 18 et 28 des coques (ou tout au moins l'un d'eux) peuvent être amincis de sorte à pouvoir se superposer partiellement d'une façon variable. Cela permet une adaptation facile du dispositif à la grosseur de la partie du corps à laquelle le dispositif doit être appliqué, et cela sans devoir couper les bords des coques. La figure 5 montre encore une feuille 33 de synderme ou matériau similaire, constituant un revêtement du dispositif et remplaçant en même temps la toile d'armature 40.

Dans la plupart des cas, la subdivision du dispositif en deux coques est suffisante, mais naturellement, lors que cela paraît nécessaire ou à propos, le dispositif peut être formé par trois coques, ou bien aussi par un nombre encore plus grand de coques.

Lors qu'il est approprié, les coques peuvent être traversées par des trous (29, figure 4) dont le diamètre, le nombre et la disposition peuvent être choisis de sorte à assurer une aération appropriée de la partie immobilisée et d'en permettre la transpiration.

Comme on peut le comprendre, l'application de l'invention offre à l'orthopédie, à la traumatologie et à la physiothérapie de réhabilitation un nouvel instrument perfectionné et, dans beaucoup de cas, très avantageux, pour le traitement des lésions de l'appareil squelettique et spécialement des fractures des membres supérieurs et inférieurs, des fractures composites, des lésions capsulo-ligamenteuses ainsi que pour des immobilisations postopératoires, pour le renouvellements d'appareils plâtrés, pour la rééducation motrice après des longues périodes de permanence au lit et pour d'autres applications.

0155730

REVENDICATIONS

1.- Dispositif de contention destiné à être appliqué à des segments lésés de l'appareil squelettique afin de les immobiliser temporairement, caractérisé en ce qu'il est formé par au moins deux coques (10,20) préformées en un matériau élastique, ayant une forme générale qui reproduit substantiellement la forme de la partie à laquelle on doit appliquer le dispositif dans le but de réaliser la contention, et prédisposées pour être assemblées au moyen de courroies (30) ou d'organes similaires de sorte qu'on puisse les ouvrir ou les enlever.

2.- Dispositif selon la revendication 1, caractérisé en ce que les coques (10,20) qui forment le dispositif sont réalisées en polyuréthane ou en un matériau synthétique expansible similaire, soumis à un dégré d'expansion approprié pour réaliser les caractéristiques de souplesse et d'élasticité générale estimées optimales.

3.- Dispositif selon la revendication 1 ou 2, caractérisé en ce qu'au moins une des coques (10,20) présente, sur sa surface extérieure, des sièges (17,27) légèrement creusés pour recevoir des courroies (30) ou des éléments similaires de connection réciproque des coques (10,20).

4.- Dispositif selon la revendication 1 ou 2, caractérisé en ce que dans la masse d'au moins une des coques (10,20), sur toute son extension ou seulement dans des endroits estimés appropriés, sont incorporés des tissus (40) ou des éléments similaires, intégrés dans la structure du matériau synthétique et formant une armature qui contribue à la stabilité de forme indépendemment de la souplesse du matériau formant la coque.

5.- Dispositif selon la revendication 1 ou 2, caractérisé en ce que dans la masse d'au moins une des coques (10,20), sur toute son extension ou seulement dans des endroits estimés appropriés, sont incorporées des

attelles (50) rigides ou semirigides s'étendant substantiellement dans la direction longitudinale de la coque (10,20).

6.- Dispositif selon la revendication 5, caractérisé en ce que lesdites attelles (50) sont formées en un matériau substantiellement radio-transparent.

7.- Dispositif selon l'une quelconque des revendications précédentes, caractérisé en ce que dans au moins une des coques (10,20) sont incorporés des matériaux de revêtement (33), tels que du synderme ou des matériaux similaires, destinés à en constituer la surface visible et éventuellement à servir aussi comme des armatures.

8.- Dispositif selon l'une quelconque des revendications précédentes, caractérisé en ce que dans au moins une des coques (10,20) sont incorporés des éléments de soutien et/ou d'articulation, destinés a des buts accéssoires.

9.- Dispositif selon l'une quelconque des revendications précédentes, caractérisé en ce qu'au moins une des coques (10,20) est traversée par des trous d'aération (29), dont le diamètre, le nombre et la disposition peuvent être variablement choisis.

10.- Dispositif selon l'une quelconque des revendications précédentes, caractérisé en ce que les coques (10,20) sont préformées selon des tailles relativement grandes et sont prédisposées pour être coupées (ligne 60) à proximité d'au moins un bord pour l'adaptation à des tailles plus minces au moyen de l'enlèvement de bandes de matériau.

11.- Dispositif selon la revendication 10, caractérisé en ce que sur au moins une des coques (10,20) sont apposées des graduations (61) destinées à faciliter la définition des lignes de coupe (60) sur la base de

mesures prises sur le patient.

12.- Dispositif selon l'une quelconque des revendications précédentes, caractérisé en ce qu'au moins un bord (18,28) d'au moins une coque
(10,20) est aminci et prédisposé pour recevoir la superposition partielle du bord (28,18) d'une autre coque (20,10).

13.- Dispositif selon l'une quelconque des revendications précédentes, caractérisé en ce que les coques (10,20) formant le dispositif sont
constructivement et fonctionnellement séparées.

14.- Dispositif selon l'une quelconque des revendication 1 à 12, caractérisé en ce que les coques (10,20) qui forment le dispostitif sont
constructivement ou au moins fonctionnellement unies, au moyen d'au
moins une partie déformable (32,19), pour leur ouverture.

0155730

FIG. 1

FIG. 2

FIG. 3

FIG. 4

FIG. 5